# EUROPÄISCHE PATENTSCHRIFT

(11) **EP 3 518 811 B1**
(45) Veröffentlichungstag und Bekanntmachung des Hinweises auf die Patenterteilung: **25.11.2020**
(21) Anmeldenummer: 17771362.5
(22) Anmeldetag: 11.09.2017
(51) Int. Cl.: A61C 1/00, A61B 17/00

(54) **FUSSPEDAL ZUM DRAHTLOSEN ANSTEUERN EINES MEDIZINISCHEN GERÄTS**
FOOT PEDAL FOR WIRELESSLY ACTUATING A MEDICAL DEVICE
PÉDALE PERMETTANT LA COMMANDE SANS FIL D'UN APPAREIL MÉDICAL

(30) Priorität: 30.09.2016 DE 102016118609
(43) Veröffentlichungstag der Anmeldung: 07.08.2019
(60) Teilanmeldung: 19208215.4
(73) Patentinhaber: Ferton Holding S.A., 2800 Delémont (CH)
(72) Erfinder: LEBRETON, Etienne, 1023 Crissier (CH); MARESCHAL DE CHARENTENAY, Antoine, 01220 Divonne les Bains (FR)
(74) Vertreter: Müller Schupfner & Partner Patent- und Rechtsanwaltspartnerschaft mbB
(86) Internationale Anmeldenummer: PCT/EP2017/072693
(87) Internationale Veröffentlichungsnummer: WO 2018/059916

(56) Entgegenhaltungen:
- US-A1- 2009 272 221
- US-A1- 2014 038 129

## Beschreibung

Die vorliegende Erfindung betrifft ein Fußpedal zum drahtlosen Ansteuern eines medizinischen Geräts, insbesondere eines zahnmedizinischen Geräts.

Solche Fußpedale sind aus dem Stand der Technik hinlänglich bekannt und sollen einem behandelndem Arzt während der Behandlung einen Handlungsspielraum gewährleisten, der es ihm erlaubt, die Behandlung fortzusetzen, ohne sie für manuelle Modifikationen am medizinischen Gerät zu unterbrechen. Statt die Behandlung zu unterbrechen, nimmt er Modifikationen am medizinischen Gerät mit Hilfe des Fußpedals vor. Wesentliche Bestandteile des Fußpedals sind ein Gehäuse mit einem Gehäuseboden und ein Deckelelement, das gegenüber dem Gehäuseboden entlang einer Betätigungseinrichtung höhenverschiebbar und neigbar gelagert ist. Typischerweise liegt das Deckelelement dabei auf einer mittig unterhalb des Deckelelements angeordneten und entlang der Betätigungsrichtung verschiebbaren Betätigungseinrichtung auf, so dass das Deckelelement zu jeder Seite neigbar, insb. verkippbar ist und sich in Betätigungsrichtung in der Regel auch zusätzlich verschieben lässt.

In der US 2009 272 221 A1 wird zur Verbesserung der drahtlosen Signalübertragung ein nicht-metallisches Deckelement aus Polycarbonat vorgeschlagen.

Aus der US 2014 038 129 A1 ist ein Fußpedal mit einer Leiterplatte zur drahtlosen Übertragung von Daten offenbart.

Hierbei erweist es sich oftmals als zweckmäßig, ein aus einem Metall gefertigtes Deckelelement zu verwenden. Neben einer vergleichsweise hohen mechanischen und chemischen Belastungsfähigkeit erweist sich die Verwendung eines metallischen Deckelelements insofern als vorteilhaft, als dass Schmutz auf dem metallischen Deckelelement schnell identifizierbar und einfach zu entfernen ist. Allerdings ist die Verwendung eines metallisches Deckelelements nachteilhaft, wenn man beabsichtigt, in das Gehäuse eine Sendeeinrichtung zu integrieren, die zur drahtlosen Kommunikation mit dem medizinischen Gerät vorgesehen ist, da das metallische Deckelelement die Sendeeinrichtung in Hinblick auf eine Signalübertragung abschirmt. Daher werden im Stand der Technik Sendeeinrichtungen üblicherweise neben das metallische Deckelelement angeordnet oder es wird ein Deckelelement aus Kunststoff verwendet. Dadurch wird nicht nur ein optischer Gesamteindruck des Fußpedals negativ beeinträchtigt, sondern das Fußpedal nimmt auch zusammen mit der Sendeeinrichtung entsprechend mehr Platz in Anspruch. Auch ist das Pedal nicht so leicht zu desinfizieren und es ist weniger stabil.

Es ist somit eine Aufgabe der vorliegenden Erfindung, ein Fußpedal bereitzustellen, das trotz eines metallischen Deckelelements kompakt ausgestaltet und zur drahtlosen Kommunikation mit dem medizinischen Gerät ausgelegt ist.

Diese Aufgabe wird gelöst durch ein Fußpedal gemäß einem der Ansprüche 1. Weitere Vorteile und Merkmale der Erfindung ergeben sich aus den Unteransprüchen sowie der Beschreibung und den beigefügten Figuren.

Erfindungsgemäß ist ein Fußpedal zum drahtlosen Ansteuern eines medizinischen Geräts, insbesondere eines zahnmedizinischen Geräts, vorgesehen, umfassend
-- ein Gehäuse mit einem Gehäuseboden,
-- ein Deckelelement, das gegenüber dem Gehäuseboden entlang einer Betätigungsrichtung höhenverschiebbar und/oder gegenüber dem Gehäuseboden neigbar gelagert ist, und
-- eine im Gehäuse angeordnete Sendeeinrichtung zur drahtlosen Kommunikation mit dem medizinischen Gerät,
wobei das Gehäuse, insbesondere der Gehäuseboden, zumindest teilweise aus einem nicht-metallischen Material und das Deckelelement aus einem metallischen Material gefertigt ist, wobei in einem Ausgangszustand das Deckelelement vom Gehäuseboden beabstandet ist und in einem abgesenkten Zustand das Deckelelement gegenüber dem Ausgangszustand in Betätigungsrichtung höhenversetzt ist, wobei das Gehäuse, insbesondere der Gehäuseboden, derart ausgestaltet ist, dass im abgesenkten Zustand ein in Betätigungsrichtung gesehen unterhalb des Deckelelements angeordneter Signalübertragungsbereich als Kommunikationskanal für ein von der Sendeeinrichtung ausgehendes Signal bereitgestellt wird.

Gegenüber dem Stand der Technik wird ein Signalübertragungsbereich unterhalb des Deckelelements bereitgestellt, der die Übertragung des Signals der Sendeeinrichtung auch dann noch sicherstellt, wenn das Deckelelement im abgesenkten Zustand ist. Infolgedessen lässt sich in vorteilhafter Weise die Sendeeinrichtung in das Gehäuse integrieren, auch wenn das Deckelelement aus einem metallischen Material gefertigt ist.

Dabei wird der Signalübertragungsbereich insbesondere durch einen Spalt zwischen dem Deckelelement und einem Fußboden, auf dem das Fußpedal angeordnet ist, gebildet. Mittels des Gehäuses bzw. des Gehäusebodens lässt sich dabei vorteilhaft im abgesenkten Zustand der kleinstmögliche Abstand für diesen Spalt realisieren, der die Signalübertragung noch sicherstellt. Vorzugsweise ist das Deckelelement im abgesenkten Zustand um den maximal möglichen Abstand zum Ausgangszustand höhenversetzt. Insbesondere wird die Wellenlänge des ausgehenden Signals bei der Größe bzw. einer Höhe des Signalübertragungsbereichs im abgesenkten Zustand berücksichtigt, d. h. der Spalt bzw. der Signalübertragungsbereich wird angepasst an die Eigenschaften des Signals der Sendeeinrichtung.

Bevorzugt liegt der Signalübertragungsbereich in Betätigungsrichtung unterhalb eines äußeren Umfangs des Deckelelements, wobei der äußere Umfang das Deckelelement in einer senkrecht zur Betätigungseinrichtung verlaufenden Schnittebene begrenzt. Beispielsweise ist der äußere Umfang als eine auf den Fußboden bzw. den Gehäuseboden gerichtete Kante bzw. ein auf den Fußboden bzw. den Gehäuseboden gerichteter Kragen ausgestaltet. Dabei ist es weiterhin vorzugsweise vorgesehen, dass im Signalübertragungsbereich das Gehäuse bzw. der Gehäuseboden aus dem nicht-metallischen Material, beispielsweise aus einem Kunststoff, gefertigt ist. Alternativ ist es auch vorstellbar, dass der Gehäuseboden im Signalübertragungsbereich eine bereichsweise Aussparung im Gehäuseboden aufweist. Weiterhin ist es bevorzugt vorgesehen, dass die Sendeeinrichtung in eine Schaltplatine, die innerhalb des Gehäuses angeordnet ist, integriert ist. Vorzugsweise ist das Deckelelement vollständig rotationssymmetrisch zu einer parallel zur Betätigungsrichtung verlaufenden Richtung und das auf der Betätigungseinrichtung gelagerte Deckelelement lässt sich zu jeder Seite hin neigen bzw. verkippen.

Gemäß einer weiteren Ausführungsform der vorliegenden Erfindung ist es vorgesehen, dass eine Erstreckung des Signalübertragungsbereichs bzw. des Spalts im abgesenkten Zustand in Betätigungsrichtung gesehen eine Höhe zwischen 1 mm und 10 mm, bevorzugt eine Höhe zwischen 1 mm und 6 mm und besonders bevorzugt im Wesentlichen eine Höhe von ca. 2-5 mm annimmt. Insbesondere wird dieser Wert an der Stelle mit der kleinsten Erstreckung bzw. Ausdehnung des Signalübertragungsbereichs in Betätigungsrichtung realisiert. Es hat sich vorteilhaft herausgestellt, dass derartig kleine Dimensionierungen für den Signalübertragungsbereich möglich sind. Entsprechend kann das Fußpedal in Betätigungsrichtung gesehen besonders kompakt ausgestaltet werden.

Vorzugsweise ist es vorgesehen, dass das Deckelelement im abgesenkten Zustand an den Gehäuseboden anschlägt. Durch das Anschlagen wird vorteilhaft dafür gesorgt, dass das Deckelelement nicht weiter abgesenkt werden kann, wodurch eine Mindesthöhe für den Signalübermittlungsbereich im abgesenkten Zustand festgelegt wird. Demnach wird verhindert, dass ein weiteres Absenken dazu führen würde, dass keine Signalübertragung mehr im abgesenkten Zustand gewährleistet werden kann. Vorzugsweise ist ein Abstand zwischen der Oberseite und der Unterseite des Gehäusebodens, im Bereich des Anschlags, zwischen 2 mm und 5 mm, bevorzugt zwischen 2 mm und 2.5 mm oder besonders bevorzugt im Wesentlichen 2 mm groß. Die Dimensionierung, bevorzugt die Dicke des jedenfalls an dieser Stelle nicht-metallischen Gehäusebodens bestimmt somit die Erstreckung bzw. Ausdehnung des Signalübertragungsbereichs an dieser, d.h. der engsten Stelle.

In einer vorteilhaften Ausführungsform der vorliegenden Erfindung ist es vorgesehen, dass die Signalübertragungseinrichtung im Bereich des äußeren Umfangs des Gehäuses angeordnet ist. Vorzugsweise umfasst das Gehäuse eine umlaufende Gehäusewandung und die Sendeeinrichtung ist an einem an die Gehäusewandung angrenzenden Bereich innerhalb des Gehäuses angeordnet. Dadurch lässt sich die Sendeeinrichtung möglichst nah an den Signalübertragungsbereich heranführen. Beispielsweise ist die Sendeeinrichtung in radialer Richtung gesehen im letzten Drittel, bevorzugt im letzten Viertel und besonders bevorzugt im letzten Fünftel einer vom Zentrum des Gehäuses zu dessen Gehäusewandung bemessenen Strecke angeordnet. Es hat sich herausgestellt, dass sich durch eine solche Anordnung die Wahrscheinlichkeit für eine erfolgreiche Signalübertragung zum medizinischen Gerät weiter verbessern lässt.

In bevorzugter Weise liegt die Sendeeinrichtung direkt am Gehäuseboden an oder ist in einem abgesenkten Teilbereich des Gehäuses eingelassen. Durch die abgesenkte Positionierung im Gehäuse lässt sich die Anordnung zur weiteren Optimierung der Signalübertragung nutzen. Dabei ist es beispielsweise vorstellbar, dass das Gehäuse topfförmig ausgestaltet ist und die Signalübertragungseinrichtung in einen Boden des Gehäuses eingelassen ist. Vorzugsweise bildet die Signalübertragungseinrichtung einen Teil des Bodens. Es ist auch vorstellbar, dass die Sendeeinrichtung im Gehäuse derart angeordnet ist, dass sie im Ausgangszustand in Betätigungsrichtung gesehen unterhalb einer unteren Kante des Deckelelements oder im Wesentlichen auf gleicher Höhe mit der unteren Kante des Deckelelements angeordnet ist.

Gemäß einer weiteren Ausführungsform der vorliegenden Erfindung ist vorgesehen, dass das von der Sendeeinrichtung ausgesendete Signal ein WIFI-Signal oder ein Bluetooth-Signal ist. Mit solchen Signalen lassen sich Kommunikationsreichweiten erzielen, die ausreichen, um mit einem mit dem Fußpedal im selben Raum angeordneten medizinischen Gerät in kommunikative Verbindung zu treten.

Vorzugsweise ist das Fußpedal zum drahtlosen Ansteuern eines medizinischen Geräts vorgesehen, wobei das Gehäuse und der Gehäuseboden über einen Magnetlösemechanismus lösbar miteinander verbunden sind. Mittels des Magnetlösemechanismus lässt sich der Gehäuseboden auf einfache und unkomplizierte Weise vom Gehäuse lösen, ohne dass beispielsweise zunächst Schrauben gelöst werden müssten. Insbesondere ist an einer Unterseite des Gehäuses eine im zusammengesetzten Zustand zum Gehäuseboden offene Aussparung vorgesehen, die zur Aufnahme von Energiespeicherzellen, insbesondere Batterien, bestimmt ist. Die Energiespeicherzellen dienen hierbei insbesondere als Energiequelle für die Sendeinrichtung. Um beim regelmäßigen Wechseln der Energiespeicherzellen den Aufwand möglichst gering zu halten, erweist sich der Magnetlösemechanismus als besonders vorteilhaft. Hierbei sind ein gehäuseseitiges Magnetelement und ein gehäusebodenseitiges Magnetelement derart ausgestaltet, dass eine zwischen den Magnetelementen wirkende magnetische Kraft die Magnetteile im zusammengesetzten Zustand des Fußpedals zusammenhält.

Vorzugsweise ist es vorgesehen, dass das Deckelelement zu einer Symmetrieachse vollständig rotationssymmetrisch ausgestaltet ist, wobei die Symmetrieachse durch den Magnetlösemechanismus verläuft oder der Magnetlösemechanismus in einer senkrecht zur Symmetrieachse verlaufenden Ebene gesehen mittig im Gehäuse angeordnet ist. Vorzugsweise ist der Magnetlösemechanismus in Betätigungsrichtung gesehen fluchtend zur Betätigungseinrichtung angeordnet. Bevorzugt bildet ein auf das Deckelelement gerichteter metallischer Vorsprung am Boden des Gehäuses das gehäuseseitige Magnetelement.

Gemäß einer weiteren Ausführungsform der vorliegenden Erfindung ist vorgesehen, dass das Fußpedal einen ersten Schalter zur Veranlassung einer ersten Funktionalität am medizinischen Gerät und einen zweiten Schalter zur Veranlassung einer zweiten Funktionalität am medizinischen Gerät aufweist, wobei der erste Schalter und der zweite Schalter mit der Sendeeinrichtung verbunden sind. Dadurch wird dem ersten Schalter und dem zweiten Schalter eine gemeinsame Sendeeinrichtung zur Verfügung gestellt und anhand einer Signalmodifikation kann dem medizinischen Gerät eine Zustandsänderung am Fußpedal mitgeteilt werden, so dass eine Steuerung über das Fußpedals und die Signalübertragungseinrichtung möglich ist.

Weiterhin ist es bevorzugt vorgesehen, dass im Gehäuseboden im Signalübertragungsbereich ein Signalverstärker zur Verstärkung des Signals von der Sendeeinrichtung integriert ist. Beispielsweise handelt es sich bei dem Signalverstärker um einen Repeater, der im Gehäuseboden im Bereich des Übertragungsbereichs dafür sorgt, dass das Signal, insbesondere das Funksignal, derart verstärkt wird, dass dem medizinischen Gerät eine möglichst große Signalgröße bereitgestellt werden kann bzw. sich eine entsprechende Reichweite mit dem Signal erzielen lässt.

Weitere Vorteile und Merkmale ergeben sich aus der nachfolgenden Beschreibung bevorzugter Ausführungsformen des erfindungsgemäßen Gegenstands mit Bezug auf die beigefügten Figuren. Einzelne Merkmale der einzelnen Ausführungsform können dabei im Rahmen der Erfindung miteinander kombiniert werden Es zeigen:
- Fig.1: eine schematische Explosionsdarstellung eines Fußpedals gemäß einer bevorzugten Ausführungsform der vorliegenden Erfindung, und
- Fig. 2a bis 2d: Schnittansichten des Fußpedals aus Fig.1 in verschiedenen Betriebszuständen.

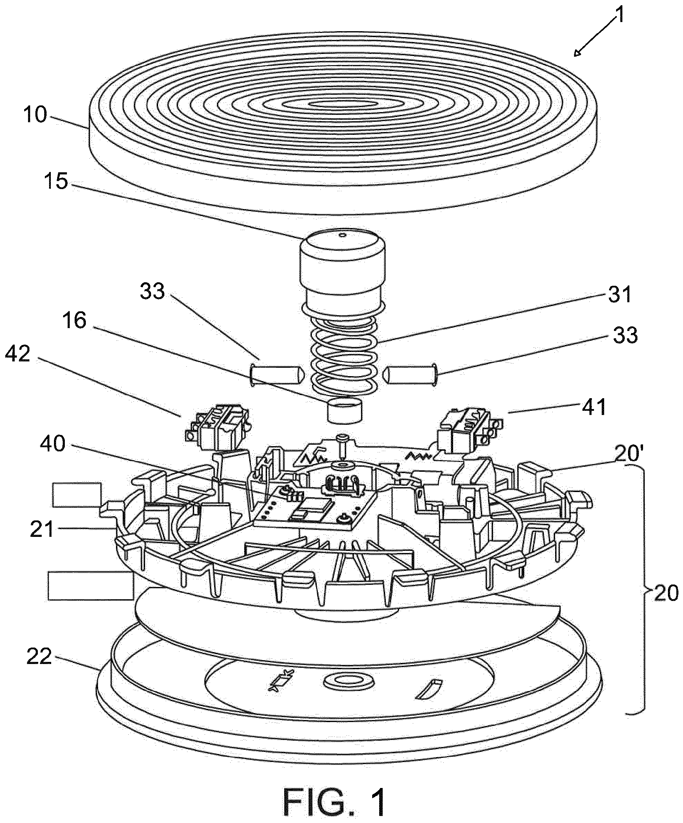

In Figur 1 ist eine schematische Explosionsdarstellung eines Fußpedals 1 gemäß einer bevorzugten Ausführungsform der vorliegenden Erfindung. Insbesondere handelt es sich bei dem Fußpedal 1 um ein solches, das zum Steuern eines medizinischen Geräts (nicht dargestellt), vorzugsweise eines zahnmedizinischen Geräts, vorgesehen ist. Dabei sollen mittels des Fußpedals 1 Einstellungen am medizinischen Gerät vorgenommen werden, so dass der Nutzer seine Arbeit, beispielsweise eine Behandlungsmaßnahme am Patienten, nicht unterbrechen muss, um das medizinische Gerät manuell zu bedienen.

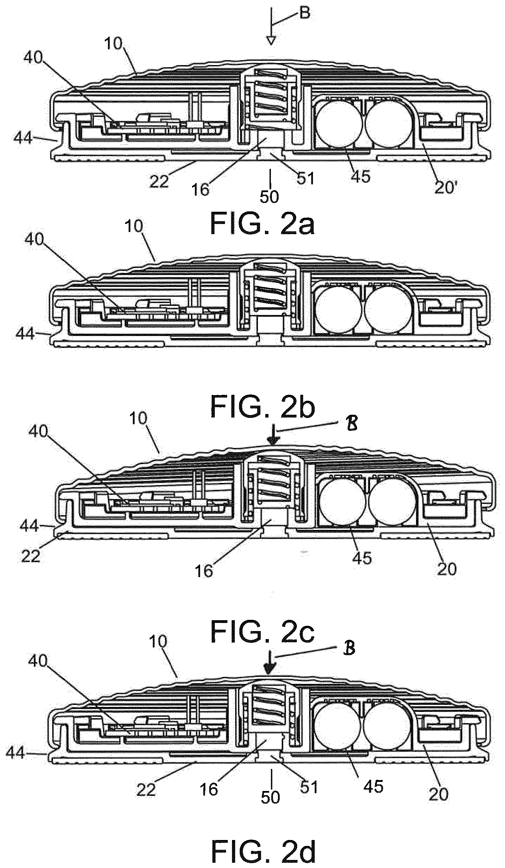

Wesentlicher Bestandteil des in der Figur 1 dargestellten Ausführungsbeispiels ist neben einem Gehäuse 20 mit einem Gehäuseboden 22 auch ein Deckelelement 10, das gegenüber dem Gehäuseboden 22 entlang einer Betätigungsrichtung B höhenverschiebbar und in diesem Fall auch neigbar gelagert ist. Konkret liegt das Deckelelement 10 hierbei zentral auf einer Betätigungseinrichtung 15 auf. Durch das zentrale Aufliegen auf der Betätigungseinrichtung 15 lässt sich das Deckelelement 10 zu allen Seiten hin verkippen bzw. neigen und durch den Kontakt zwischen dem Deckelelement 10 und der Betätigungseinrichtung 15 wird die Betätigungseinrichtung 15 in Betätigungsrichtung B verschoben, wenn eine Krafteinwirkung, insbesondere von einem Fuß ausgehende Krafteinwirkung, auf das Deckelelement 10 in vertikaler Richtung erfolgt. Dabei kann ein Höhenversatz der Betätigungseinrichtung sowohl beim Auftreten auf den Randbereich als auch beim Auftreten auf den Zentralbereich erfolgen. Der Höhenversatz der Betätigungseinrichtung beim Auftreten auf den Randbereich wird dabei dadurch unterstützt, dass beim Verkippen die Bewegung des Deckelelements auf der dem betätigten Randbereich gegenüberliegenden Seite nach oben, d. h. in einer der Betätigungsrichtung entgegengesetzten Richtung, begrenzt ist. Um die Betätigungseinrichtung 15 wieder in ihren Ausgangszustand zu überführen, ist eine Feder 31 vorgesehen, die zur einen Seite an den Gehäuseboden 22 bzw. einen Gehäusegrundkörper 20' (Fig. 2) und zur anderen Seite an die Betätigungseinrichtung 15 angebunden ist.

Abhängig von Position und Größe der Krafteinwirkung unterscheiden sich dabei unterschiedliche Höhenversätze, die durch ein Auftreten mit dem Fuß auf das Deckelelement 10 veranlasst werden. Wirkt beispielsweise eine Kraft auf den Randbereich des Deckelelements 10, wird das Deckelelement 10 um einen durch die Betätigungseinrichtung 15 bereitgestellten Hebelpunkt bzw. eine von der Betätigungseinrichtung bereitgestellte Hebelfläche verkippt. Neben der Neigung wird zusätzlich ein Höhenversatz der höhenverschiebbar gelagerten Betätigungseinrichtung 15 veranlasst. Dieser Höhenversatz ist allerding kleiner als ein solcher, der durch eine Kraft mit derselben Größe veranlasst wird, wenn die Kraft oberhalb der Betätigungseinrichtung 15 und parallel zur Betätigungsrichtung 15 auf das Deckelelement 10, d.h. in diesem Beispiel im Wesentlichen vertikal einwirkt. Entsprechend lassen sich die unterschiedlichen Höhenversätze zur gezielten Differenzierung zwischen zwei Arten der Betätigung des Fußpedals 1 nutzen, wodurch den unterschiedlichen Betätigungsarten verschiedene Funktionalitäten zugeordnet werden können. Insbesondere lässt sich einem Auftreten auf den Randbereich und dem dadurch bewirkten Verkippen des Deckelelements 10 eine erste Funktionalität und einem Auftreten auf den Zentralbereich des Deckelelements 10 eine zweite Funktionalität zuordnen.

Um diesen Höhenversatzunterschied bei den unterschiedlichen Betätigungen zur Differenzierung der Betätigungen mit dem Fuß auszunutzen, sind insbesondere ein erster Schalter 41 und ein zweiter Schalter 42 vorgesehen, die zueinander höhenversetzt angeordnet sind, wobei der erste Schalter 41 bei einer Bewegung der Betätigungseinrichtung 15 in Betätigungsrichtung B innerhalb eines ersten Höhenversatzabschnitts und der zweite Schalter 42 innerhalb eines zweiten Höhenversatzabschnitts betätigbar sind. Dabei schließt sich der zweite Höhenversatzabschnitt in Betätigungsrichtung B unmittelbar an den ersten Höhenversatzabschnitt an. Die Betätigung des ersten Schalters 41 und des zweiten Schalters 42 erfolgt beispielsweise über senkrecht zur Betätigungsrichtung B verschiebbare Stifte 33, die mittels Federelementen (nicht dargestellt) gegen eine Außenseite der Betätigungseinrichtung 15 vorgespannt sind. Die Außenseite der Betätigungseinrichtung 15 weist dabei eine Außenkontur, beispielsweise in Gestalt eines Vorsprungs bzw. einer Rampe, auf. Diese Außenkontur wirkt bei der Bewegung der Betätigungseinrichtung 15 in Betätigungsrichtung B mit den verschiebbaren Stiften 33 so zusammen, dass die Stifte 33 zur Seite weg, d. h. entlang einer senkrecht zur Betätigungsrichtung B verlaufenden Richtung und entgegen der Rückstallkraft der Federelemente, radial nach außen gedrückt werden, wodurch der erste Schalter 41 bzw. zweite Schalter 42 betätigt werden können. Indem der erste Schalter 41 und der zweite Schalter 42 höhenversetzt zueinander angeordnet sind, können der erste Schalter 42 und der zweite Schalter 42 durch eine in Betätigungsrichtung B verlaufende Bewegung der Betätigungseinrichtung 15 nacheinander, insbesondere abhängig vom jeweiligen Höhenversatz der Betätigungseinrichtung 15, geschaltet werden und zwar unabhängig von der Position der Kraft, die auf den Randbereich des insbesondere kreisrunden Deckelelements wirkt.

Zur drahtlosen Kommunikation mit dem medizinischen Gerät ist eine Sendeeinrichtung 40 vorgesehen, die innerhalb des Gehäuses 20 angeordnet ist und die mit dem ersten Schalter 41 und dem zweiten Schalter 42 verbunden ist. Vorzugsweise sendet die Sendeinrichtung 40 zur Kommunikation mit dem medizinischen Gerät ein WIFI-Signal oder ein Bluetooth-Signal aus. Versorgt wird die Sendeeinrichtung hierbei über eine innerhalb des Gehäuses 20 angeordnete Energiespeicherzelle 45, z. B. eine Batterie. Weiterhin ist es vorgesehen, dass das Deckelelement 10 aus einem metallischen Material gefertigt ist, so dass eine Signalübertragung über das Deckelelement 10 ausgeschlossen ist. Mit anderen Worten: das Deckelelement 10 schirmt die Sendeeinrichtung 40 ab. Eine Übertragung des Signals erfolgt entsprechend über einen Signalübertragungsbereich 44, der zur einen Seite durch das Deckelelement 10 und zur anderen Seite durch einen Fußboden, auf dem das Fußpedal 1 angeordnet ist, begrenzt ist.

In den Figuren 2a bis 2d ist das Fußpedal 1 aus Figur 1 in verschiedenen Betriebszuständen dargestellt. In Figur 2a befindet sich das Deckelelement 10 in einem Ausgangszustand. In diesem Ausgangszustand wirkt keine äußere Kraft auf das Deckelelement 10 und die Feder 31 beabstandet das Deckelelement 10 zum Gehäuseboden 22. In Betätigungsrichtung B gesehen wird somit unterhalb des Deckelelements 10, konkret zwischen dem Deckelelement 10 und einem Fußboden, auf dem das Fußpedal 1 angeordnet ist, ein ausreichend großer Spalt gebildet, der als Signalübertragungsbereich 44 dient.

In den Figuren 2b und 2c ist das Deckelelement 10 jeweils geneigt bzw. verkippt dargestellt, wobei in Figur 2c das Deckelelement 10 jedenfalls teilweise in Anlage mit dem Gehäuseboden 22 kommt. Dabei wird jedenfalls auf der Seite, die einem Bereich, in dem das Deckelelement 10 mit dem Gehäuseboden 22 in Anlage ist, in senkrecht zur Betätigungsrichtung verlaufenden Richtung gegenüberliegt, ein Spalt gebildet, der ausreichend groß für eine Signalübertragung ist. In der Figur 2d ist das Deckelelement 10 in einem abgesenkten Zustand dargestellt, in dem das Deckelelement 10 mit seinem Kragen am äußeren Umfang umlaufend am Gehäuseboden 22 anliegt. Hier ist es vorgesehen, dass der Gehäuseboden 22 derart ausgestaltet ist, dass auch im abgesenkten Zustand ein Signalübertragungsbereich 44 bereitgestellt wird.

In der in den Figuren 2a bis 2d dargestellten Ausführungsform ist der Gehäuseboden 22 derart ausgestaltet, dass das abgesenkte Deckelelement 10 an eine Oberseite des Gehäusebodens 22, insbesondere an einen umlaufenden Absatz am Gehäuseboden 22, anschlägt. Der Abstand zwischen der Oberseite und einer der Oberseite in Betätigungsrichtung gegenüberliegenden Unterseite des Gehäusebodens 22 ist dabei derart dimensioniert, dass der Gehäuseboden 22 einen Signalübertragungsbereich 44 im abgesenkten Zustand bereitstellt. Hierzu ist der Gehäuseboden 22 insbesondere in einem zwischen der Sendeeinrichtung und einem äußeren Umfang des Gehäusebodens liegenden Bereich ausgespart und/oder aus einem nichtmetallischen Material gefertigt, um eine Signalfortsetzung sicherzustellen. Vorzugsweise ist ein Abstand zwischen der Oberseite und der Unterseite des Gehäusebodens 22 zwischen 2 mm und 5 mm, bevorzugt zwischen 2 mm und 2.5 mm oder besonders bevorzugt im Wesentlichen 2 mm groß.

Weiterhin ist es vorgesehen, dass das Gehäuse 20 bzw. ein Gehäusegrundkörper 20' eine umlaufende Gehäusewand 21 (Fig. 1) aufweist, wobei die Sendeeinrichtung 40 in einem an die Gehäusewandung 21 angrenzenden Bereich innerhalb des Gehäuses 20 angeordnet ist. Um die Signalübertragung durch den Gehäuseboden 22 weiter zu unterstützen, ist die Sendeeinrichtung in einem abgesenkten Bereich des Gehäuses 20 bzw. des Gehäusegrundkörpers 20' angeordnet.

Weiterhin ist es vorzugsweise vorgesehen, dass das Gehäuse 20 als Einsatz in Form eines Gehäusegrundkörpers 20' ausgestaltet ist, der an den Gehäuseboden 22 angebunden ist. Vorzugsweise wird der Gehäuseboden 22 hierbei über einen Magnetlösemechanismus 50 an das Gehäuse 20 gebunden. Hierzu umfasst der Gehäuseboden 22 einen Magneten 51, der mit einem magnetischen Teil des Gehäuses 20 bzw. des Gehäusegrundkörpers 20' zusammenwirkt. Vorzugsweise ist der Magnetlösemechanismus 50 unterhalb der Betätigungseinrichtung 15 angeordnet. Insbesondere bildet ein Vorsprung 16, an den die Betätigungseinrichtung 15 bei der Bewegung entlang der Betätigungsrichtung B in Anlage gerät, den magnetischen Teil des Gehäuses 20, der den Gehäuseboden 22 mit dessen Magneten 51 im zusammengebauten Zustand festhält.

Der Magnetlösemechanismus 50 erlaubt ein unkompliziertes Lösen des Gehäusebodens 22 vom Gehäuse 20. Weiterhin ermöglicht der Magnetlösemechanismus 50, sofern er, wir in den Figuren dargestellt, zentral am Gehäuseboden 22 angebracht ist, eine Rotationsbewegung des Gehäusegrundkörpers 20' und des mit diesem verbundenen Deckelelements 10 um den Gehäuseboden 22, der in der Regel haftend mit dem Fußboden verbunden ist. Hierzu sind die sich gegenüberstehenden Oberflächen des Magneten 51 und des magnetischen Teils des Gehäuses 20, wie z.B. des Vorsprungs 16, derart gelagert, dass eine rotierende Bewegung ermöglicht wird. Bevorzugt sind glatte Oberflächen, die aufeinander gleiten.

Vorzugsweise ist das Gehäuse an seiner dem Gehäuseboden 22 zugewandten Seite derart ausgestaltet, dass ein Fach für Energiespeicherzellen 45 frei zugänglich ist, wenn der Gehäuseboden 22 vom Gehäuse 20 getrennt wird. Zusammen mit dem Magnetlösemechanismus 50 lässt sich ein Wechsel der Energiespeicherzellen 45 vereinfachen, der für die Versorgung der Sendeeinrichtung 40 im Gehäuse regelmäßig erforderlich ist.

### Bezugszeichenliste

- 1: Fußpedal
- 10: Deckelelement
- 15: Betätigungseinrichtung
- 16: Vorsprung
- 20: Gehäuse
- 20': Gehäusegrundkörper
- 21: Gehäusewandung
- 31: Feder
- 33: Stift
- 40: Sendeeinrichtung
- 41: erster Schalter
- 42: zweiter Schalter
- 44: Signalübertragungsbereich
- 45: Energiespeicherzelle
- 50: Magnetlösemechanismus
- 51: Magnet
- B: Betätigungsrichtung

## Patentansprüche

1. Fußpedal (1) zum drahtlosen Ansteuern eines medizinischen Geräts, insbesondere eines zahnmedizinischen Geräts, umfassend
-- ein Gehäuse (20) mit einem Gehäuseboden (22),
-- ein Deckelelement (10), das gegenüber dem Gehäuseboden (22) entlang einer Betätigungsrichtung (B) höhenverschiebbar und/oder gegenüber dem Gehäuseboden (22) neigbar gelagert ist, und
-- eine im Gehäuse (20) angeordnete Sendeeinrichtung (40) zur drahtlosen Kommunikation mit dem medizinischen Gerät,
wobei in einem Ausgangszustand das Deckelelement (10) vom Gehäuseboden (22) beabstandet ist und in einem abgesenkten Zustand das Deckelelement (10) gegenüber dem Ausgangszustand in Betätigungsrichtung (B) höhenversetzt ist, wobei
das Gehäuse (20), insbesondere der Gehäuseboden (22), derart ausgestaltet ist, dass im abgesenkten Zustand ein in Betätigungsrichtung (B) gesehen unterhalb des Deckelelements (10) angeordneter Signalübertragungsbereich (44) als Kommunikationskanal für ein von der Sendeeinrichtung (40) ausgehendes Signal bereitgestellt wird, **dadurch gekennzeichnet, dass** das Gehäuse 20, insbesondere der Gehäuseboden (22), zumindest teilweise aus einem nicht-metallischen Material und das Deckelelement (10) aus einem metallischen Material gefertigt ist.

2. Fußpedal (1) gemäß Anspruch 1, **dadurch gekennzeichnet, dass** eine Erstreckung des Signalübertragungsbereichs (44) im abgesenkten Zustand in Betätigungsrichtung gesehen eine Höhe zwischen 1 mm und 10 mm, bevorzugt eine Höhe zwischen 1 mm und 6 und besonders bevorzugt im Wesentlichen eine Höhe von 2 -5 mm annimmt.

3. Fußpedal (1) gemäß einem der vorhergehenden Ansprüche, wobei das Deckelelement (10) im abgesenkten Zustand an den Gehäuseboden (22) anschlägt.

4. Fußpedal (1) gemäß einem der vorhergehenden Ansprüche, wobei die Sendeeinrichtung (40) im Bereich des äußeren Umfangs des Gehäuses (20) angeordnet ist.

5. Fußpedal (1) gemäß einem der vorhergehenden Ansprüche, wobei die Sendeeinrichtung (40) direkt am Gehäuseboden (22) anliegt oder in einem abgesenkten Teilbereich des Gehäuses (22) eingelassen ist.

6. Fußpedal (1) gemäß einem der vorhergehenden Ansprüche, wobei das von der Sendeeinrichtung (40) ausgesendete Signal ein WIFI-Signal oder ein Bluetooth-Signal ist.

7. Fußpedal (1) gemäß einem der vorhergehenden Ansprüche, **dadurch gekennzeichnet, dass** das Gehäuse (20) und der Gehäuseboden (22) über einen Magnetlösemechanismus (50) lösbar miteinander verbunden sind.

8. Fußpedal (1) gemäß Anspruch 7, **dadurch gekennzeichnet, dass** das Deckelelement (10) zu einer Symmetrieachse vollständig rotationssymmetrisch ausgestaltet ist und wobei die Symmetrieachse durch den Magnetlösemechanismus (50) verläuft oder der Magnetlösemechanismus (50) in einer senkrecht zur Symmetrieachse verlaufenden Schnittebene gesehen mittig im Gehäuse (20) angeordnet ist.

9. Fußpedal (1) gemäß einem der vorhergehenden Ansprüche, **dadurch gekennzeichnet, dass** das Fußpedal (1) einen ersten Schalter (41) zur Veranlassung einer ersten Funktionalität am medizinischen Gerät und einen zweiten Schalter (42) zur Veranlassung einer zweiten Funktionalität am medizinischen Gerät aufweist, wobei der erste Schalter (41) und der zweite Schalter (42) mit der Sendeeinrichtung (40) verbunden sind.

10. Fußpedal (1) gemäß einem der vorhergehenden Ansprüche, wobei im Gehäuseboden (22) im Signalübertragungsbereich (44) ein Signalverstärker zur Verstärkung des Signals von der Sendeeinrichtung (40) integriert ist.

## Claims

1. A foot pedal (1) for wireless control of a medical device, in particular a dental device, comprising
-- a housing (20) with a housing base (22),
-- a cover element (10), which is mounted so as to be vertically displaceable relative to the housing base (22) along an actuating direction (B) and/or inclinable relative to the housing base (22), and
-- a transmitting device (40) arranged in the housing (20) for wireless communication with the medical device,
wherein in an initial state the cover element (10) is spaced apart from the housing base (22) and in a lowered state the cover element (10) is offset in height with respect to the initial state in the operating direction (B), wherein
the housing (20), in particular the housing base (22), is configured such that, in the lowered state, a signal transmission region (44) arranged below the cover element (10), viewed in the actuating direction (B), is provided as a communication channel for a signal emitted from the transmitting device (40), **characterized in that**
the housing, in particular the housing base (22), is at least partially made of a non-metallic material and the cover element (10) is made of a metallic material.

2. The foot pedal (1) according to claim 1, **characterized in that** an extension of the signal transmission region (44) in the lowered state, viewed in the actuating direction, has a height between 1 mm and 10 mm, preferably a height between 1 mm and 6 and more preferably essentially a height of 2 - 5 mm.

3. The foot pedal (1) according to one of the preceding claims, wherein the cover element (10) comes in abutment with the housing base (22) in the lowered state.

4. The foot pedal (1) according to one of the preceding claims, wherein the transmitting device (40) is disposed in the region of the outer periphery of the housing (20).

5. The foot pedal (1) according to one of the preceding claims, wherein the transmitting device (40) is attached directly on the housing base (22) or is embedded in a lowered portion of the housing (22).

6. The foot pedal (1) according to one of the preceding claims, wherein the signal transmitted by the transmitting device (40) is a WIFI signal or a Bluetooth signal.

7. The foot pedal (1) according to one of the preceding claims, **characterized in that** the housing (20) and the housing base (22) are detachably connected to one another by means of a magnetic release mechanism (50).

8. The foot pedal (1) according to claim 7, **characterized in that** the cover element (10) is configured to be completely rotationally symmetrical with respect to an axis of symmetry, and wherein the axis of symmetry runs through the magnet release mechanism (50), or the magnet release mechanism (50) is arranged centrally in the housing (20) as seen in a section plane running perpendicularly to the axis of symmetry.

9. The foot pedal (1) according to one of the preceding claims, **characterized in that** the foot pedal (1) has a first switch (41) for initiating a first functionality on the medical device and a second switch (42) for initiating a second functionality on the medical device, wherein the first switch (41) and the second switch (42) are connected to the transmitting device (40).

10. The foot pedal (1) according to one of the preceding claims, wherein a signal amplifier for amplifying the signal from the transmitting device (40) is integrated in the housing base (22) in the signal transmission region (44).

## Revendications

1. Pédale (1) destinée à la commande sans fil d'un appareil médical, en particulier d'un appareil de médecine dentaire, incluant
- un boîtier (20) avec un fond du boîtier (22),
- un élément formant couvercle (10), qui est déplaçable en hauteur par rapport au fond du boîtier (22) le long d'une direction d'actionnement (B) et/ou qui est monté de façon à pouvoir être incliné par rapport au fond du boîtier (22), et
- un dispositif émetteur (40) agencé dans le boîtier (20) pour une communication sans fil avec l'appareil médical,
dans lequel, dans un état de départ, l'élément formant couvercle (10) est à distance du fond du boîtier (22) et, dans un état abaissé, l'élément formant couvercle (10) et déplacé en hauteur en direction d'actionnement (B) par rapport à l'état de départ, dans laquelle
le boîtier (20), en particulier le fond de boîtier (22), est conçu de telle façon que dans l'état abaissé une zone de transmission de signal (44) agencée au-dessous de l'élément formant couvercle (10), vue dans la direction d'actionnement (B), est mise à disposition à titre de canal de communication pour un signal partant du dispositif émetteur (40),
**caractérisée en ce que** le boîtier (20), en particulier le fond du boîtier (22) est réalisé au moins partiellement en un matériau non métallique et l'élément formant couvercle (10) est réalisé en un matériau métallique.

2. Pédale (1) selon la revendication 1, **caractérisée en ce qu'**une extension de la zone de transmission de signal (44) dans l'état abaissé, occupe une hauteur, vue en direction d'actionnement, entre 1 mm et 10 mm, de préférence entre 1 mm et 6, et de façon particulièrement préférée une hauteur essentiellement de 2 à 5 mm.

3. Pédale (1) selon l'une des revendications précédentes, dans laquelle l'élément formant couvercle (10), dans l'état abaissé, vient buter contre le fond du boîtier (22).

4. Pédale (1) selon l'une des revendications précédentes, dans laquelle le dispositif émetteur (40) est agencé dans la région de la périphérie extérieure du boîtier (20).

5. Pédale (1) selon l'une des revendications précédentes, dans laquelle le dispositif émetteur (40) est appliqué directement contre le fond du boîtier (22) ou est intégré dans une zone partielle surbaissée du fond du boîtier (22).

6. Pédale (1) selon l'une des revendications précédentes, dans laquelle le signal émis par le dispositif émetteur (40) et un signal WIFI ou un signal Bluetooth.

7. Pédale (1) selon l'une des revendications précédentes, **caractérisée en ce que** le boîtier (20) et le fond du boîtier (22) sont reliés l'un à l'autre de façon détachable via un mécanisme de déclenchement magnétique (50).

8. Pédale (1) selon la revendication 7, **caractérisée en ce que** l'élément formant couvercle (10) est conçu entièrement à symétrie de révolution par rapport à un axe de symétrie et dans laquelle l'axe de symétrie s'étend à travers le mécanisme de déclenchement magnétique (50), ou **en ce que** le mécanisme de déclenchement magnétique (50) est agencé au milieu dans le boîtier (20), vu dans un plan de coupe s'étendant perpendiculairement à l'axe de symétrie.

9. Pédale (1) selon l'une des revendications précédentes, **caractérisée en ce que** la pédale (1) comprend un premier commutateur (41) pour déclencher une première fonctionnalité au niveau de l'appareil médical et un second commutateur (42) pour déclencher une seconde fonctionnalité au niveau de l'appareil médical, le premier commutateur (41) et le second commutateur (42) étant connectés au dispositif émetteur (40).

10. Pédale (1) selon l'une des revendications précédentes, dans laquelle un amplificateur de signal est intégré dans le fond de boîtier (22) dans la zone de transmission de signal (44), afin d'amplifier le signal du dispositif émetteur (40).
